# EUROPEAN PATENT APPLICATION

(11) **EP 4 700 134 A1**
(43) Date of publication of application: **25.02.2026**
(21) Application number: 24807558.2
(22) Date of filing: 16.05.2024
(51) Int. Cl.: C12Q 1/6883

(54) **NOVEL BOVINE TUBERCULOSIS BIOMARKER AND USE THEREOF**

(30) Priority: 16.05.2023 KR 20230063402; 14.05.2024 KR 20240063402
(71) Applicant: Xenohelix. Co., Ltd., Incheon 21984 (KR)
(72) Inventor: CHO, Seok Keun, Incheon 21982 (KR); RYU, Moon Young, Incheon 21982 (KR); WOO, Jin Kyu, Bucheon-si, Gyeonggi-do 14548 (KR)
(74) Representative: Stolmár & Partner Patentanwälte PartG mbB
(86) International application number: PCT/KR2024/006624
(87) International publication number: WO 2024/237687

(57) **Abstract**

The present invention relates to novel biomarkers for diagnosing bovine tuberculosis, a diagnostic or prognostic composition comprising the same, a kit comprising the same, and the use thereof.

The novel biomarkers of the present invention and/or the compositions comprising the same may enable rapid and accurate diagnosis and/or prognosis of bovine tuberculosis, in which infection and onset are difficult to determine due to the absence of distinct symptoms.
In particular, since the biomarkers of the present invention exhibit high sensitivity and specificity, they can provide great utility not only in the diagnosis and/or prognosis of bovine tuberculosis but also in the screening of therapeutic agents for bovine tuberculosis.

## Description

### TECHNICAL FIELD

The present invention relates to novel biomarkers for bovine tuberculosis, diagnostic or prognostic compositions comprising the biomarkers, kits comprising the compositions, and uses thereof.

### BACKGROUND ART

Bovine tuberculosis, as a zoonotic disease, is rapidly transmitted, directly or indirectly, from cattle with pulmonary lesions or from heavy shedders such as those with tuberculous mastitis. In individuals with severe pulmonary tuberculous lesions or in cases that have developed systemic infection, symptoms such as coughing, loss of luster of the hair coat, loss of appetite, loss of vigor, decreased milk yield, and emaciation are observed; however, there are also many cases in which no abnormal external findings are present and diagnosis is made only after necropsy.

Diagnosis of bovine tuberculosis includes the purified protein derivative (PPD) tuberculin skin test as an intradermal test, and diagnostic methods using immune cell culture such as the lymphocyte transformation test (LTT); as methods to confirm mycobacterial infection, isolation and identification of the tubercle bacilli, PCR, and HPLC are employed; and ELISA interferon-gamma diagnostic kits utilizing antibody-detection methods against the tubercle bacilli (bovine tuberculosis antibody ELISA) are generally used.

However, bovine tuberculosis is a disease that can be transmitted by animals showing no obvious clinical symptoms, and because early diagnosis is difficult, culling of infected animals upon detection is employed as a reliable control measure. In particular, since wild animals also serve as reservoirs of tuberculosis, it is necessary to control the transmission of the disease from wildlife to livestock.

Under these circumstances, the continuous development of technologies for the detection or diagnosis of bovine tuberculosis is required, and in particular, there is an ongoing need for the identification of biomarkers and the development of diagnostic technologies that enable early and accurate diagnosis for timely response to tuberculosis.

### DESCRIPTION OF EMBODIMENTS

### TECHNICAL PROBLEM

The object of the present invention is to provide a biomarker composition for diagnosing or determining prognosis of bovine tuberculosis.

Another object of the present invention is to provide a composition for diagnosing or determining prognosis of bovine tuberculosis.

A further object of the present invention is to provide a kit for diagnosing or determining prognosis of bovine tuberculosis, comprising the aforementioned diagnostic or prognostic composition.

A still further object of the present invention is to provide a method of providing information for diagnosing or determining prognosis of bovine tuberculosis.

Another object of the present invention is to provide a method for diagnosing bovine tuberculosis.

A further object of the present invention is to provide a method for screening therapeutic agents for bovine tuberculosis.

### TECHNICAL SOLUTION

One aspect of the present invention relates to a biomarker composition for the diagnosis or prognosis of bovine tuberculosis, comprising one or more genes selected from the group consisting of RUBCNL (Rubicon like autophagy enhancer), CXCL1 (Chemokine (C-X-C motif) ligand 1), CXCL2 (Chemokine (C-X-C motif) ligand 2), SMIM7 (Small Integral Membrane Protein 7), CTSW (Cathepsin W), TAC3 (Tachykinin Precursor 3), IL2 (Interleukin 2), and NRADD (Neurotrophin receptor associated death domain protein).

In one embodiment, the RUBCNL may consist of the nucleotide sequence of SEQ ID NO: 1, the CXCL1 may consist of the nucleotide sequence of SEQ ID NO: 2, the CXCL2 may consist of the nucleotide sequence of SEQ ID NO: 3, the SMIM7 may consist of the nucleotide sequence of SEQ ID NO: 4, the CTSW may consist of the nucleotide sequence of SEQ ID NO: 5, the TAC3 may consist of the nucleotide sequence of SEQ ID NO: 6, the IL2 may consist of the nucleotide sequence of SEQ ID NO: 7, and the NRADD may consist of the nucleotide sequence of SEQ ID NO: 8.

In the present invention, "Bovine Tuberculosis", also referred to as "pearl disease," is a chronic wasting zoonotic infectious disease. It is caused by *Mycobacterium bovis* (*M. bovis*), and the main routes of infection are small droplets contained in the cough of infected animals or feed contaminated with saliva. It can also be transmitted through the ingestion of unpasteurized milk, as well as through feces or placenta. Because the disease progresses slowly and the symptoms are not distinct, it is difficult to identify infected animals, and therefore, infected animals without symptoms often transmit the disease to other cohabiting animals.

In the present invention, the term "biomarker" refers to an indicator that can measure biological changes in a living organism using DNA, RNA, proteins, or metabolites, and through this, it is possible to measure whether a disease has developed, whether an infection has occurred, and the degree of drug response in a living organism.

In the present invention, the term "gene" refers to a factor that expresses the genetic traits of an organism and may include nucleic acid materials composed of bases or modified bases such as DNA or RNA.

In an exemplary embodiment of the present invention, it was confirmed that the newly identified biomarkers RUBCNL, CXCL1, CXCL2, SMIM7, CTSW, TAC3, IL2, and/or NRADD exhibited high AUC values of 0.8 or 0.9 or higher in ROC analysis, indicating that they can exhibit very high accuracy.

Another aspect of the present invention relates to a composition for the diagnosis or prognosis of bovine tuberculosis, comprising an agent for measuring the expression level or detecting the presence of a mutation of one or more gene expression products selected from the group consisting of RUBCNL, CXCL1, CXCL2, SMIM7, CTSW, TAC3, IL2, and NRADD.

In one embodiment, the gene expression product may be an mRNA, a protein, or a fragment thereof encoded by one or more genes selected from the group consisting of RUBCNL, CXCL1, CXCL2, SMIM7, CTSW, TAC3, IL2, and NRADD, but is not limited thereto.

In the present invention, the term "agent" may refer to one or more selected from the group consisting of sense or antisense primers, probes, aptamers, antibodies, peptides, and nucleotides that specifically bind to a gene expression product, but is not limited thereto.

The term "primer" refers to a short nucleic acid sequence having a free 3' hydroxyl group, which can form base pairs with a complementary template and functions as a starting point for replication of the template strand. The primer allows the initiation of DNA synthesis in the presence of appropriate buffer and temperature conditions, reagents for polymerization reactions (i.e., DNA polymerase or reverse transcriptase), and different nucleoside triphosphates. The PCR conditions, as well as the length of the sense and antisense primers, may be modified based on those known to a person skilled in the art.

The term "probe" refers to a nucleic acid fragment such as RNA or DNA, ranging in length from a few bases to several tens of bases. The probe may be prepared in the form of an oligonucleotide probe, a single-stranded DNA (ssDNA) probe, a double-stranded DNA (dsDNA) probe, or an RNA probe. The DNA may include cDNA, genomic DNA, or oligonucleotides; the RNA may include genomic RNA, mRNA, or oligonucleotides; and the protein may include antibodies, antigens, enzymes, or peptides. The selection of an appropriate probe, the hybridization conditions, and whether or not labeling is performed may be modified based on those known to a person skilled in the art.

The term "aptamer" refers to a single-stranded nucleic acid (DNA, RNA, or modified nucleic acid) molecule having a tertiary structure and capable of binding to a target molecule with high affinity and specificity. Various modifications can be made to the nucleic acid material, and by binding to the gene expression product of the present invention to allow measurement of its expression level, it can be applied for diagnostic or prognostic purposes.

The term "antibody" refers to a specific protein molecule that is directed against an antigenic site. The antibody may refer to a protein molecule that specifically binds to a bovine tuberculosis biomarker according to one embodiment, and may include polyclonal antibodies, monoclonal antibodies, or immunoglobulin antibodies, as well as special antibodies such as humanized antibodies. In addition, the antibody may include not only a complete form having two full-length light chains and two full-length heavy chains, but also functional fragments of the antibody molecule. The functional fragment of the antibody molecule refers to a fragment that retains at least antigen-binding activity, and examples thereof include Fab, F(ab'), F(ab')₂, and Fv.

The term "peptide" refers to all forms comprising an amino acid sequence capable of specifically binding to a gene expression product, and the length, structure, and labeling of the peptide may be appropriately modified and applied as needed.

The term "nucleotide" refers to all forms including nucleic acids such as RNA, DNA, or PNA that can complementarily bind to a gene expression product, and the length, structure, and labeling of the nucleotide may be appropriately modified and applied as needed.

In the present invention, the term "diagnosis" includes determining the susceptibility of a subject, i.e., an object to be tested, to a specific disease or disorder; determining whether a subject currently has a specific disease or disorder; determining the prognosis of a subject suffering from a specific disease or disorder; or therametrics (for example, monitoring the condition of a subject to provide information regarding therapeutic efficacy).

In the present invention, the term "prognosis" means to anticipate or estimate in advance a medical outcome, and refers to presuming in advance the progression, improvement, recurrence, recurrence after drug treatment, or survival of a disease.

Another aspect of the present invention relates to a composition for diagnosing or determining prognosis of bovine tuberculosis, comprising the bovine tuberculosis diagnostic or prognostic biomarker composition described above. The composition may include, together with the bovine tuberculosis diagnostic or prognostic biomarker composition comprising one or more genes selected from the group consisting of RUBCNL, CXCL1, CXCL2, SMIM7, CTSW, TAC3, IL2, and NRADD, a carrier, excipient, additive, or an agent capable of measuring the expression of the biomarker gene, as necessary, but is not limited thereto.

Another aspect of the present invention relates to a kit for diagnosing or determining prognosis of bovine tuberculosis, comprising the above-described composition for diagnosis or prognosis of bovine tuberculosis.

The kit of the present invention can be used to diagnose the onset of bovine tuberculosis by measuring the expression level of a biomarker gene in blood, serum, urine, or the like of an individual suspected of having bovine tuberculosis, and may include, without limitation, components, solutions, or devices necessary for measurement or analysis, such as primers, probes, or antisense nucleotides capable of measuring the expression level of the gene. For example, the kit may include an RT-PCR (Reverse Transcription Polymerase Chain Reaction) kit, a DNA chip kit, a microarray chip kit, or a protein chip kit, but is not limited thereto.

The diagnostic kit may include a solution, a lyophilized powder, a frozen solution, or a strip form, and each form may be formulated by conventional methods known in the art. In addition, the kit may include an instruction manual for use.

Another aspect of the present invention relates to a method for providing information for diagnosing or determining prognosis of bovine tuberculosis, the method comprising: a) measuring the expression level of one or more genes selected from the group consisting of RUBCNL, CXCL1, CXCL2, SMIM7, CTSW, TAC3, IL2, and NRADD in a sample isolated from an individual; and b) comparing the expression level of the gene with the corresponding gene expression level in a normal control sample.

In one embodiment, the method may further include a step of determining bovine tuberculosis when the expression level of RUBCNL, CXCL1, or CXCL2 is decreased compared with that of the normal control.

In one embodiment, the method may further include a step of determining bovine tuberculosis when the expression level of SMIM7, CTSW, TAC3, IL2, or NRADD is increased compared with that of the normal control.

In the present invention, the sample may be blood, tissue, cells, whole blood, plasma, serum, saliva, sputum, lymphatic fluid, cerebrospinal fluid, intercellular fluid, or urine, and as long as it corresponds to a sample applicable to the diagnosis of bovine tuberculosis, it is not limited to the above examples and may be applied as a sample.

Another aspect of the present invention relates to a method for diagnosing bovine tuberculosis, the method comprising: a) measuring the expression level of one or more genes selected from the group consisting of RUBCNL, CXCL1, CXCL2, SMIM7, CTSW, TAC3, IL2, and NRADD in a sample isolated from an individual; and b) comparing the expression level of the gene with the corresponding gene expression level in a normal control sample.

In one embodiment, the method may further include a step of determining bovine tuberculosis when the expression level of RUBCNL, CXCL1, or CXCL2 is decreased compared with that of the normal control.

In one embodiment, the method may further include a step of determining bovine tuberculosis when the expression level of SMIM7, CTSW, TAC3, IL2, or NRADD is increased compared with that of the normal control.

In the diagnostic method of the present invention, the sample may be blood, tissue, cells, whole blood, plasma, serum, saliva, sputum, lymphatic fluid, cerebrospinal fluid, intercellular fluid, or urine, and as long as it corresponds to a sample applicable to the diagnosis of bovine tuberculosis, it is not limited to the above examples and may be applied as a sample.

In addition, another aspect of the present invention relates to a method for screening a therapeutic agent for bovine tuberculosis, the method comprising:
a) treating a sample with a candidate substance for a bovine tuberculosis therapeutic agent; and
b) measuring the expression level of one or more genes selected from the group consisting of RUBCNL, CXCL1, CXCL2, SMIM7, CTSW, TAC3, IL2, and NRADD in the sample treated with the candidate substance.

In one embodiment, the method may further include a step of determining the treated candidate substance as a therapeutic agent for bovine tuberculosis when the expression level of RUBCNL, CXCL1, or CXCL2 measured in step b) is increased compared with that in the sample not treated with the candidate substance.

In one embodiment, the method may further include a step of determining the treated candidate substance as a therapeutic agent for bovine tuberculosis when the expression level of SMIM7, CTSW, TAC3, IL2, or NRADD measured in step b) is decreased compared with that in the sample not treated with the candidate substance.

In an exemplary embodiment of the present invention, it was confirmed that RUBCNL, CXCL1, CXCL2, SMIM7, CTSW, TAC3, IL2, and/or NRADD were increased or decreased in bovine tuberculosis-infected cattle compared with normal cattle, and through ROC analysis, it was confirmed that when these genes are used as biomarkers, bovine tuberculosis-infected cattle can be distinguished with high sensitivity and accuracy. Accordingly, when the expression of RUBCNL, CXCL1, or CXCL2, which had been decreased, is increased following treatment with a candidate substance, or when the expression of SMIM7, CTSW, TAC3, IL2, or NRADD, which had been increased, is decreased, the candidate substance can be selected as a therapeutic agent for bovine tuberculosis.

### ADVANTAGEOUS EFFECTS OF INVENTION

The novel biomarkers of the present invention and/or the compositions comprising the same may enable rapid and accurate diagnosis and/or prognosis of bovine tuberculosis, in which infection and onset are difficult to determine due to the absence of distinct symptoms.
In particular, since the biomarkers of the present invention exhibit high sensitivity and specificity, they can provide great utility not only in the diagnosis and/or prognosis of bovine tuberculosis but also in the screening of therapeutic agents for bovine tuberculosis.
The effects of the present invention are not limited to the above-described effects, and it should be understood to include all effects that can be inferred from the configuration of the invention described in the detailed description or the claims of the present invention.

### BRIEF DESCRIPTION OF THE DRAWINGS

FIG. 1 shows gene biomarkers whose expression was increased by comparing normal cattle and bovine tuberculosis-infected cattle.
FIG. 2 shows a comparison of expression levels and ROC analysis results of RUBCNL and CXCL1, which exhibited changes in expression by comparing normal cattle and bovine tuberculosis-infected cattle.
FIG. 3 shows a comparison of expression levels and ROC analysis results of CXCL2, SMIM7, and TAC3, which exhibited changes in expression by comparing normal cattle and bovine tuberculosis-infected cattle.
FIG. 4 shows the results of ROC analysis for the IL2, CTSW, and NRADD biomarkers.

### DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Example 1. Extraction and purification of RNA from bovine blood samples

Blood samples were collected from bovine tuberculosis-negative and -positive cattle whose infection status with *Mycobacterium bovis* had been confirmed using the PPD intradermal test and the ELISA interferon-gamma diagnostic kit. Total RNA was extracted from the collected blood samples using the XENOPURE^{™} Total RNA Purification Kit (XENOHELIX, Cat. No. 93667873-TR).

Specifically, 750 µl of XENOPURE^{™}-TR Lysis Buffer was added to 250 µl of a blood sample, mixed by vortexing for 1 minute, and then centrifuged at 12,300 × g for 1 minute. The supernatant was transferred to a new 1.5 ml microcentrifuge tube. To this, 200 µl of chloroform was added and mixed thoroughly by vortexing for 30 seconds. The mixture was allowed to stand at room temperature for 3 minutes and then centrifuged at 12,300 × g for 10 minutes at 4 °C.
Three hundred microliters of the upper aqueous phase was transferred to a DNA removal column and centrifuged at 12,300 × g for 1 minute. The column containing the bound genomic DNA was discarded, and the flow-through collected in the collection tube was transferred to a new 1.5 ml microcentrifuge tube. An equal volume of binding solution-Total RNA (BS-TR) was added to the tube containing the flow-through and mixed gently.

The mixed solution was added to a XENOPURE^{™} RNA column fitted with a 2 ml collection tube and centrifuged at 2,000 × g for 1 minute at room temperature, after which the flow-through collected in the collection tube was discarded. Subsequently, 700 µl of Washing Buffer 1 (WB 1) was added to the XENOPURE^{™} RNA column and centrifuged at 2,000 × g for 1 minute at room temperature, and the flow-through collected in the collection tube was discarded.
Next, 700 µl of Washing Buffer 2 (WB 2) was added to the XENOPURE^{™} RNA column and centrifuged at 2,000 × g for 1 minute at room temperature, and the flow-through collected in the collection tube was discarded. Again, 700 µl of Washing Buffer 3 (WB 3) was added to the XENOPURE^{™} RNA column and centrifuged at 2,000 × g for 1 minute at room temperature, and the flow-through collected in the collection tube was discarded.

Thereafter, the XENOPURE^{™} RNA column was placed into a new 2 ml collection tube, and with the cap of the column open, centrifugation was performed at 12,300 × g for 3 minutes to dry the membrane. The collection tube and the flow-through were discarded. The XENOPURE^{™} RNA column was then placed into a new 1.5 ml microcentrifuge tube, and 40 µl of Elution Buffer (EB) was added onto the membrane of the XENOPURE^{™} RNA column. After closing the cap of the column, centrifugation was carried out at 12,300 × g for 2 minutes to obtain total RNA.

### Example 2. cDNA Synthesis

The RNA obtained in Example 1 was quantified using a Nanodrop instrument, and 100 ng was placed into a tube. The XENO cDNA Synthesis Kit-TB (XENOHELIX, Cat. No. 9366CTB) was used. Subsequently, 5 µl of XENO 4X RT Reaction Mix-TB and 1 µl of XENO RTase Mix were added, and finally, nuclease-free water was added to bring the total reaction volume to 20 µl. After thorough mixing, the reaction was carried out at 25°C for 5 minutes, 50°C for 20 minutes, and 95°C for 1 minute, followed by cooling at 4°C to generate cDNA strands. The synthesized cDNA product was stored frozen at -20°C.

### Example 3. qPCR Procedure

From the cDNA synthesized in Example 2, 2 µl of cDNA, 1 µl of a target gene-specific forward primer (10 pmol/µl), 1 µl of a reverse primer (10 pmol/µl), 0.5 µl of a target gene-specific probe (10 pmol/µl), 12.5 µl of qPCR mixture (XENOHELIX, Cat. No. 9366XPM), and 8 µl of DW were mixed to prepare a final reaction volume of 25 µl. The mixture was added to a 96-well qPCR plate, and the qPCR reaction was performed. The Bio-Rad CFX Connect Real-Time PCR Detection System was used under the following PCR conditions: one cycle at 95°C for 5 minutes, followed by 40 cycles at 95°C for 15 seconds and 63°C for 1 minute. Based on the Cq values obtained using the CFX Maestro qPCR Analysis Software, the relative expression levels of the target genes were calculated.

The primer and probe sequences used in the present invention are as follows. β-actin was used as a reference gene for comparison of gene expression.

**[Table 1]**

| Gene | Primer/Probe | Sequence (5'-3') | SEQ ID NO. |
|---|---|---|---|
| RUBCNL | Forward primer | AGGATCACAGCGAGGAGAA | 9 |
| | Reverse primer | GGCATGTTGCATGGTCTTTC | 10 |
| | Probe | 5'FAM-AAAGTCTACCCTCCGCAGCAACAT-3'BHQ1 | 11 |
| CXCL1 | Forward primer | CATCGATAAGATGCTAAACAAGGC | 12 |
| | Reverse primer) | ACTGAGGCTGCTGGAGTA | 13 |
| | Probe | 5`FAM-TGCCAACTGATCAAGAGAGATGGAACG- 3`BHQ1 | 14 |
| CXCL2 | Forward primer | CCGCTCCCATGGTTAAGAAA | 15 |
| | Reverse primer | GTCTACTTCTGGAACAGCCATC | 16 |
| | Probe | 5'FAM-TAGGCCAGCTCTAACTGACCAGGT-3'BHQI | 17 |
| SMIM7 | Forward primer | AGCACAGGTGACAACATCC | 18 |
| | Reverse primer | AGCACGATCATGCAGAACA | 19 |
| | Probe | 5'FAM-TCATCGCCCTGTGGAATGTCTTCA-3'BHQ1 | 20 |
| CTSW | Forward primer | GGAAAGTTGGCACCATCTCA | 21 |
| | Reverse primer | GTCTGAACTTGATGGCCCATAG | 22 |
| | Probe | 5'FAM-CAACAGTTGCAGTTTCGCTGGTCC-3'BHQ1 | 23 |
| TAC3 | Forward primer | CCTGGATGGATTGCTCAAGAT | 24 |
| | Reverse primer | CCCATGAGACCCACAAAGAA | 25 |
| | Probe | 5'FAM-AGCGTAGGTCCTAAGGAGTCACCA-3'BHQI1 | 26 |
| IL2 | Forward primer | TGCACCTACTTCAAGCTCTAC | 27 |
| | Reverse primer | AGCTTGAGGTTCTCAGGATTT | 28 |
| | Probe | 5`FAM-AGCAACTGTAAATCCAGCAGCAATGAC- 3`BHQ1 | 29 |
| NRADD | Forward primer | GTTGCTCTGTGGTGTGAGTT | 30 |
| | Reverse primer | ATGCCAGGAAGCCCATAAAG | 31 |
| | Probe | 5'FAM-TCTCAGGGAACCTGCTCTGGTG-3'BHQ1 | 32 |
| β -actin | Forward primer | CTTCCAGCAGATGTGGATCAG | 33 |
| | Reverse primer | AAGGGTGTAACGCAGCTAAC | 34 |
| | Probe | 5'FAM-TCGTCCACCGCAAATGCTTCTAGG-3"BHQ1 | 35 |

### Example 4. Identification of Biomarkers for Diagnosis and Prognosis of Bovine Tuberculosis

### 4-1. Discovery of Bovine Tuberculosis Biomarkers

Through the procedures of Examples 1 to 3, novel biomarkers capable of detecting bovine tuberculosis were discovered by identifying genes whose expression was decreased or increased in blood samples from bovine tuberculosis-infected cattle compared with normal cattle.

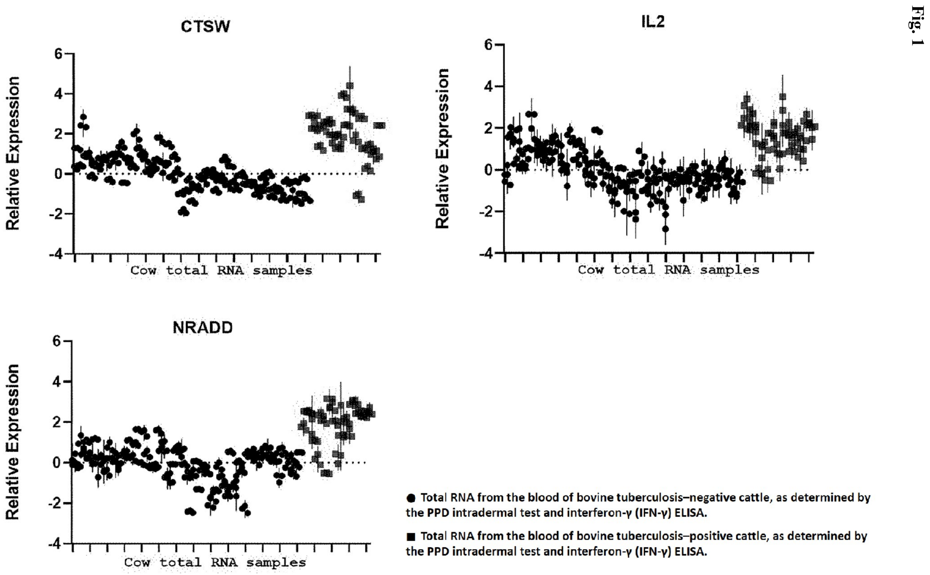

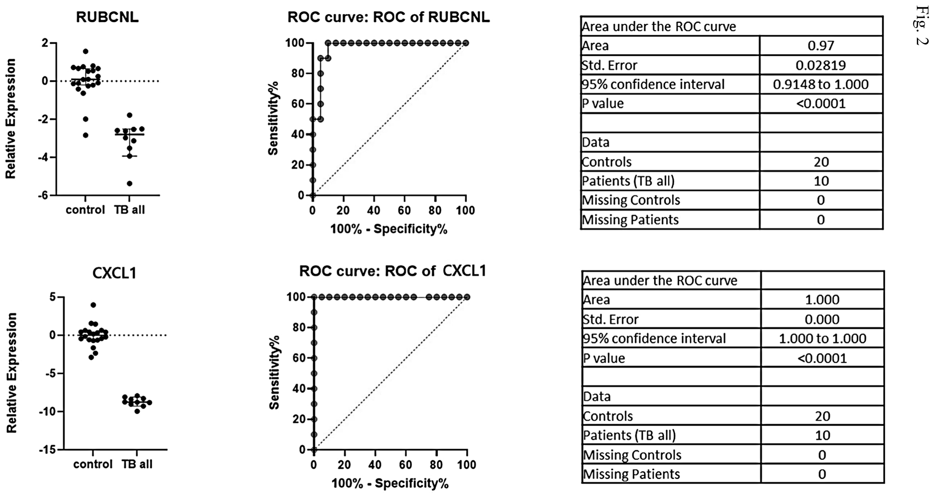

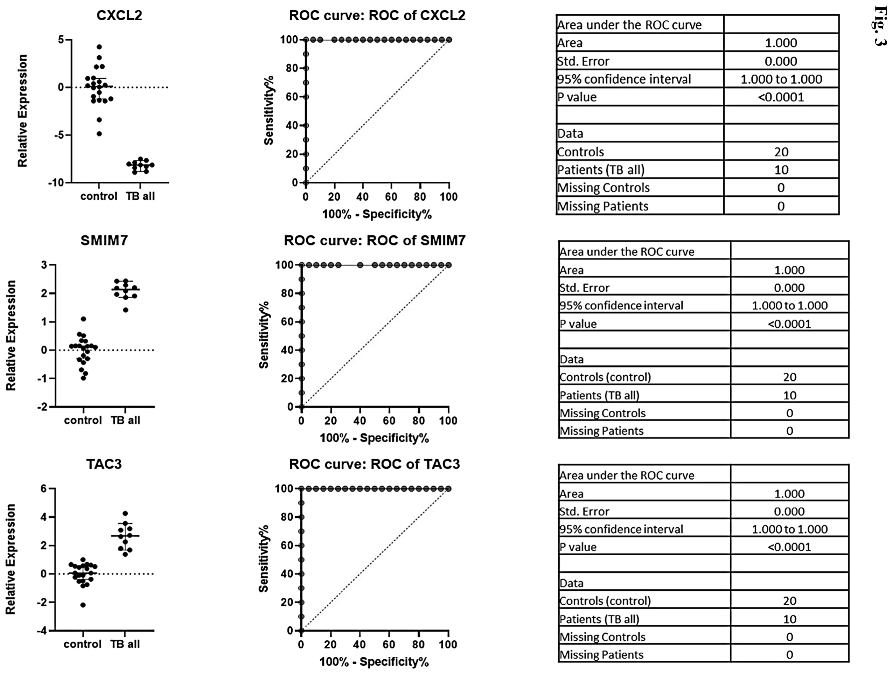

For example, as shown in FIG. 1, the genes CTSW, IL2, and NRADD, whose expression levels were significantly increased in bovine tuberculosis-infected cattle compared with normal cattle, were selected. In addition, as shown in FIGS. 2 and 3, RUBCNL, CXCL1, and CXCL2, whose expression levels were decreased in bovine tuberculosis-infected cattle compared with normal cattle, were selected, and SMIM7 and TAC3, whose expression levels were increased in bovine tuberculosis-infected cattle compared with normal cattle, were also selected.

The selected bovine tuberculosis biomarkers are shown in Table 2 below.

**[Table 2]**

| GENE | Full name | Ref Seq. | SEQ ID NO. |
|---|---|---|---|
| RUBCNL | Rubicon like autophagy enhancer | NM_001102041 | 1 |
| CXCL1 | Chemokine (C-X-C motif) ligand 1 / GRO1 | NM_175700 | 2 |
| CXCL2 | Chemokine (C-X-C motif) ligand 2 / GRO2 | NM_174299 | 3 |
| SMIM7 | Small Integral Membrane Protein 7 | NM_001205376 | 4 |
| CTSW | Cathepsin W (Lymphopain) | NM_001110070 | 5 |
| TAC3 | Tachykinin Precursor 3 | NM_181017 | 6 |
| IL2 | Interleukin 2 | NM_180997 | 7 |
| NRADD | Neurotrophin receptor associated death domain protein | NM_001076398 | 8 |

### 4-3. Verification of Bovine Tuberculosis Biomarkers

To verify the sensitivity and specificity of the biomarkers discovered in section 4-1, the area under the ROC (receiver operating characteristic) curve (AUC) value was determined. The ROC analysis is a graph that represents the correlation between sensitivity and specificity in a specific diagnostic method and can indicate the accuracy of a given diagnostic model.

In the present invention, "sensitivity" refers to the proportion of individuals who actually have the disease and are correctly determined as positive when using a specific diagnostic model, and "specificity" refers to the proportion of individuals who do not have the disease and are correctly determined as negative when using a specific diagnostic model.

In such ROC analysis, when both specificity and sensitivity are high, the accuracy of the test increases. For example, assuming the total area is 1, the accuracy can be considered higher when the AUC is 0.5 or higher, 0.6 or higher, 0.7 or higher, 0.8 or higher, or 0.9 or higher, and the closer the curve in the ROC graph is to the upper left corner, the higher the accuracy can be considered. In addition, in the ROC curve, the larger the area under the curve (AUC), the higher the accuracy of the corresponding diagnostic model can be considered.

As a result, as shown in FIG. 2, RUBCNL and CXCL1 were confirmed to have decreased expression compared with normal cattle and to exhibit high AUC values of 0.9 or higher, indicating high accuracy as biomarkers.

Furthermore, as shown in FIG. 3, CXCL2 was found to have decreased expression compared with normal cattle and exhibited an exceptionally high AUC value close to 1.0, confirming high accuracy as a biomarker. In addition, SMIM7 and TAC3 exhibited increased expression compared with normal cattle and also showed exceptionally high AUC values close to 1.0, confirming high accuracy as biomarkers.

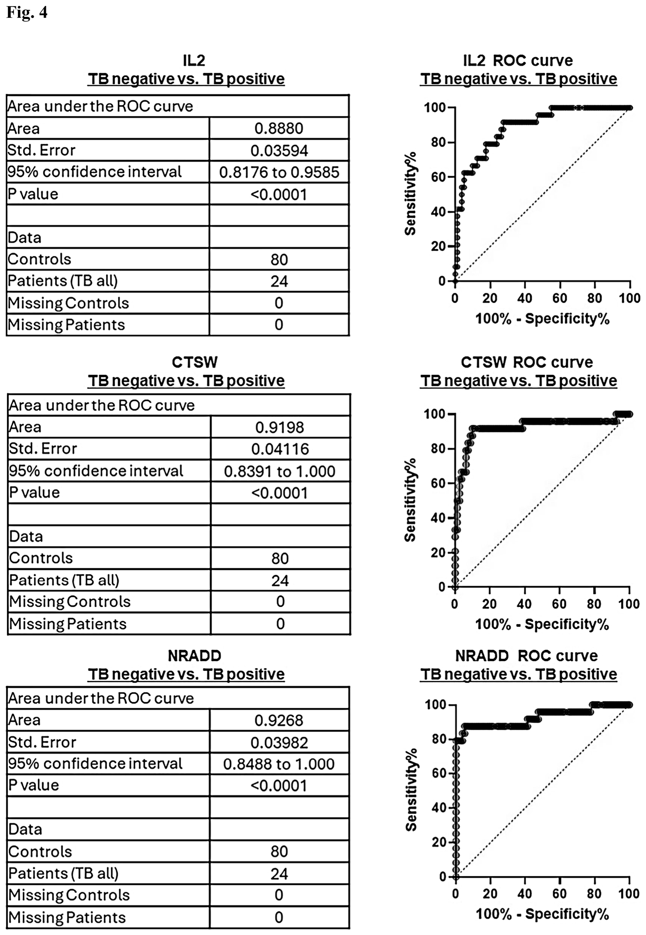

Furthermore, as shown in FIG. 4, IL2 exhibited a high AUC value of 0.8 or higher, and CTSW and NRADD exhibited high AUC values of 0.9 or higher, confirming high accuracy as biomarkers.

These results demonstrate that the biomarkers discovered in the present invention have extremely high specificity and sensitivity for bovine tuberculosis, and thus can detect and diagnose bovine tuberculosis with very high accuracy.

The biomarkers may be applied individually or in combination to meaningfully diagnose bovine tuberculosis, and even in cases of bovine tuberculosis with no distinct clinical symptoms, the occurrence of the disease can be predicted based solely on gene expression information.

All statistical analyses in the present invention were performed using analysis of variance (ANOVA) to evaluate differences among groups, and diagnostic values for each biomarker were calculated by analyzing the receiver operating characteristic (ROC) curve and the area under the ROC curve (AUC). Using the Wilson/Brown method, which is one of the methods for estimating confidence intervals, the optimal statistical cutoff value was calculated based on the ROC curve, and sensitivity, specificity, accuracy, positive predictive value, and negative predictive value for the selected cutoff point were evaluated using the generalized estimating equation method.
All statistical analyses of the present invention were performed using GraphPad Prism 9.5.1 (GraphPad Software, Boston, MA, USA), and a p-value of less than 0.0001 was considered statistically significant.

The foregoing description of the present invention is for illustrative purposes, and it will be understood by those skilled in the art to which the present invention pertains that various modifications can be made in other specific forms without departing from the technical spirit or essential characteristics of the present invention. Therefore, the embodiments described above should be understood as illustrative in all respects and not restrictive. For example, each component described as being in a singular form may be implemented in a distributed manner, and likewise, components described as being distributed may be implemented in a combined form.

The scope of the present invention is defined by the following claims, and all modifications or variations derived from the meaning and scope of the claims and their equivalents shall be construed as being included within the scope of the present invention.

## Claims

1. A bovine tuberculosis diagnostic or prognostic biomarker composition comprising one or more genes selected from the group consisting of RUBCNL (Rubicon-like autophagy enhancer), CXCL1 (Chemokine (C-X-C motif) ligand 1), CXCL2 (Chemokine (C-X-C motif) ligand 2), SMIM7 (Small Integral Membrane Protein 7), CTSW (Cathepsin W), TAC3 (Tachykinin Precursor 3), IL2 (Interleukin 2), and NRADD (Neurotrophin receptor associated death domain protein).

2. The bovine tuberculosis diagnostic or prognostic biomarker composition of claim 1,
wherein the RUBCNL consists of the nucleotide sequence of SEQ ID NO: 1,
wherein the CXCL1 consists of the nucleotide sequence of SEQ ID NO: 2,
wherein the CXCL2 consists of the nucleotide sequence of SEQ ID NO: 3,
wherein the SMIM7 consists of the nucleotide sequence of SEQ ID NO: 4,
wherein the CTSW consists of the nucleotide sequence of SEQ ID NO: 5,
wherein the TAC3 consists of the nucleotide sequence of SEQ ID NO: 6,
wherein the IL2 consists of the nucleotide sequence of SEQ ID NO: 7, and
wherein the NRADD consists of the nucleotide sequence of SEQ ID NO: 8.

3. A composition for diagnosing or determining prognosis of bovine tuberculosis, comprising an agent for measuring the expression level or detecting the presence of a mutation of one or more gene expression products selected from the group consisting of RUBCNL, CXCL1, CXCL2, SMIM7, CTSW, TAC3, IL2, and NRADD.

4. The composition for diagnosing or determining prognosis of bovine tuberculosis of claim 3, wherein the gene expression product is an mRNA, a protein, or a fragment thereof encoded by one or more genes selected from the group consisting of RUBCNL, CXCL1, CXCL2, SMIM7, CTSW, TAC3, IL2, and NRADD.

5. The composition for diagnosing or determining prognosis of bovine tuberculosis of claim 3, wherein the agent is one or more selected from the group consisting of sense or antisense primers, probes, aptamers, antibodies, peptides, and nucleotides that can specifically bind to a gene expression product.

6. A composition for diagnosing or determining prognosis of bovine tuberculosis, comprising the biomarker composition of claim 1.

7. A kit for diagnosing or determining prognosis of bovine tuberculosis, comprising the composition of any one of claims 3 to 6.

8. A method for providing information for diagnosing or determining prognosis of bovine tuberculosis, comprising:
a) measuring the expression level of one or more genes selected from the group consisting of RUBCNL, CXCL1, CXCL2, SMIM7, CTSW, TAC3, IL2, and NRADD in a sample isolated from an individual; and
b) comparing the expression level of the gene with the corresponding gene expression level in a normal control sample.

9. The method of claim 8, further comprising a step of determining bovine tuberculosis when the expression level of RUBCNL, CXCL1, or CXCL2 is decreased compared with that of the normal control.

10. The method of claim 8, further comprising a step of determining bovine tuberculosis when the expression level of SMIM7, CTSW, TAC3, IL2, or NRADD is increased compared with that of the normal control.

11. The method of claim 8, wherein the sample is blood, tissue, cells, whole blood, plasma, serum, saliva, sputum, lymphatic fluid, cerebrospinal fluid, intercellular fluid, or urine.

12. A method for screening a therapeutic agent for bovine tuberculosis, comprising:
a) treating a sample with a candidate substance for a bovine tuberculosis therapeutic agent; and
b) measuring the expression level of one or more genes selected from the group consisting of RUBCNL, CXCL1, CXCL2, SMIM7, CTSW, TAC3, IL2, and NRADD in the sample treated with the candidate substance.

13. The method of claim 12, further comprising a step of determining the treated candidate substance as a therapeutic agent for bovine tuberculosis when the expression level of RUBCNL, CXCL1, or CXCL2 measured in step b) is increased compared with that in the sample not treated with the candidate substance.

14. The method of claim 12, further comprising a step of determining the treated candidate substance as a therapeutic agent for bovine tuberculosis when the expression level of SMIM7, CTSW, TAC3, IL2, or NRADD measured in step b) is decreased compared with that in the sample not treated with the candidate substance.

15. A method for diagnosing bovine tuberculosis, comprising:
a) measuring the expression level of one or more genes selected from the group consisting of RUBCNL, CXCL1, CXCL2, SMIM7, CTSW, TAC3, IL2, and NRADD in a sample isolated from an individual; and
b) comparing the expression level of the gene with the corresponding gene expression level in a normal control sample.

16. The method of claim 15, further comprising a step of determining bovine tuberculosis when the expression level of RUBCNL, CXCL1, or CXCL2 is decreased compared with that of the normal control.

17. The method of claim 15, further comprising a step of determining bovine tuberculosis when the expression level of SMIM7, CTSW, TAC3, IL2, or NRADD is increased compared with that of the normal control.

18. The method of claim 15, wherein the sample is blood, tissue, cells, whole blood, plasma, serum, saliva, sputum, lymphatic fluid, cerebrospinal fluid, intercellular fluid, or urine.
